(19) Europäisches Patentamt
European Patent Office
Office européen des brevets

(11) Publication number: 0 075 337
B1

(12) **EUROPEAN PATENT SPECIFICATION**

(45) Date of publication of patent specification: **23.10.85**

(21) Application number: **82200974.2**

(22) Date of filing: **28.07.82**

(51) Int. Cl.⁴: **C 07 C 51/12,** C 07 C 53/08, C 07 C 53/122, C 07 C 53/124, C 07 C 63/06, C 07 C 53/02, C 07 C 67/36, C 07 C 67/37, C 07 C 69/14, C 07 C 69/24, C 07 C 69/16

# (54) Process for the co-production of carboxylic acids and carboxylic acid esters.

(30) Priority: **22.09.81 GB 8128606**

(43) Date of publication of application:
**30.03.83 Bulletin 83/13**

(45) Publication of the grant of the patent:
**23.10.85 Bulletin 85/43**

(84) Designated Contracting States:
**BE DE FR GB IT NL**

(56) References cited:
**EP-A-0 031 606**
**GB-A-1 450 993**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 075 337 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for the co-production of carboxylic acids and carboxylic acid esters from carboxylic acid esters having one carbon atom less in the molecule, carbon monoxide and hydrogen in the presence of a catalytic system. The invention relates in particular to a process for the co-production of acetic acid and ethyl acetate from methyl acetate under mild process conditions. Carboxylic acid esters produced according to the process according to the present invention are thus homologues of the carboxylic acid esters used as starting materials.

The production of carboxylic acid esters via homologation has already been described in the literature. For instance, it is known from Dutch published patent application 7807520 that carboxylic acid esters (especially ethyl acetate from methyl acetate) can be prepared at elevated temperatures at a pressure of at least 100 bar, preferably between 200 and 1500 bar in the presence of cobalt, rhodium, ruthenium, or iron or salts thereof. Apart from the fact that very high pressures have to be applied in order to get a reasonable conversion, the process as described also has the disadvantage that water is produced as the co-product. It will be clear that water can cause hydrolysis of the esters present in the reaction mixture (whether being present as product or as starting material). Even when very high, unattractive pressures (e.g. well over 1000 bar) are used substantial amounts of alkanols are produced.

It is further known from German Offenlegungsschrift 2733663 that the homologation of methyl acetate (or of its precursor dimethyl ether) can be carried out using a ruthenium carbonyl compound and an iodide or bromide promotor at elevated temperatures and pressures. The process as described in the German Offenlegungsschrift also has to be carried out at very high pressures (well over 200 bar) and substantial amounts of other products (including not only alkanols but also methane) are formed.

It is known from Dutch published patent application 7602096 that methyl acetate can be converted using carbon monoxide and hydrogen in the presence of a specific catalyst comprising a Group VIII noble metal compound and a halogen (especially iodine) source into acetic acid and ethylidene diacetate. However, ethyl acetate (the homologation product according to the process according to the present invention) is not even mentioned as a by-product.

European Patent Application No. 31606 discloses that by the use of a very specific catalytic system, the stoichiometry of the known reactions of methylacetate with carbon monoxide and hydrogen can be altered most advantageously to produce one mole of ethyl acetate and two moles of acetic acid from two moles of methyl acetate. The catalytic system comprises three metal compounds: a ruthenium compound, a further Group VIII metal compound, and a bromide or iodide of a Group II or transition metal. As is common in catalytic systems, the reaction mixture preferably also contains a promotor, typically an amine, such as alpha-picoline, or a phosphine, such as triphenylphosphine. Further said specification teaches that the homologation of methyl acetate over a catalytic system comprising ruthenium III chloride trihydrate and palladium acetate, is most successful if zinc iodide is present in the catalyst system and Comparative Example D of said specification shows that the reaction is not successful if the zinc iodide is omitted.

Most surprisingly, it has now been found that an alkyl or acyl iodide or bromide as a component in the catalytic system without the presence of zinc iodide enhances the production of ethyl acetate and acetic acid, provided that the reaction is carried out in the presence of a phosphine in an amount not exceeding a certain level.

The present invention provides a process for the co-production of carboxylic acids of the general formula $R^1$—COOH and $R^2$—COOH and carboxylic acid esters of the general formula $R^1$—$COOCH_2R^2$ and $R^2$—$COOCH_2R^1$ wherein each of the groups $R^1$ and $R^2$, which may be the same or different, represents an alkyl group having from 1 to 20 carbon atoms which may be substituted by fluorine or chlorine or an aryl, alkaryl or aralkyl group which may be substituted by fluorine or chlorine whilst $R^1$ may also represent a hydrogen atom, in which process a carboxylic acid ester of the general formula $R^1$—$COOR^2$ and/or an ether of the general formula $R^3OR^4$, wherein $R^1$ and $R^2$ are as defined hereinbefore and each of $R^3$ and $R^4$, which may be the same or different, represents an alkyl group having from 1 to 20 carbon atoms which may be substituted by fluorine or chlorine or an aryl, alkaryl or aralkyl group which may be substituted by fluorine or chlorine is reacted with carbon monoxide and hydrogen at elevated temperature and pressure characterized in that the reaction is carried out in the presence of a catalytic system which comprises a ruthenium compound, a further Group VIII metal compound except iron, nickel and cobalt, a phosphine and a compound of the general formula $R^5Hal$ or $R^5COHal$ where $R^5$ has one of the meanings given above for $R^2$ and Hal represents an iodine or bromine atom, the reaction mixture being substantially free from Group II metal iodides or bromides, and containing not more than 2 mol of phosphine per gram atom of ruthenium.

It should be noted that the composition of the reaction product mixture will be governed by the choice of the starting carboxylic acid esters and/or ethers. For instance, when starting materials are used wherein the groups $R^1$ and $R^2$ are identical, such as in methyl acetate, dimethyl ether and ethyl propionate the reaction product mixture will normally contain only the carboxylic acid ester homologue and the appropriate acid. When starting materials are used wherein the groups $R^1$

and $R^2$ are not identical, a more complex reaction product mixture will be obtained which comprises normally at least two carboxylic acid ester homologues and two appropriate carboxylic acids. For instance, when ethyl acetate is used as the starting material the reaction product mixture comprises propyl acetate, ethyl propionate, propionic acid and acetic acid.

It will be appreciated that any carboxylic acid ester homologue produced according to the present process can serve as starting material in the process according to the present invention thus forming the next carboxylic acid ester homologue(s) and the appropriate carboxylic acid(s). In addition, since carboxylic acids are produced in the process according to the present invention, transesterification reactions, i.e. reactions between carboxylic acids and carboxylic acid esters, or between different carboxylic acid esters, may also occur under the prevailing reaction conditions. It will be clear that transesterification reactions do not alter the product composition when the starting material comprises compounds wherein $R^1$ and $R^2$ are identical, but may alter the product composition when the groups $R^1$ and $R^2$ are not identical.

For the purpose of the present invention, carboxylic acids and carboxylic acid esters, obtained via a further homologation of produced carboxylic acid ester, or obtained by a transesterification process under the prevailing conditions, are considered to be within the scope of the present invention.

From the above it will be clear that preference is given to processes wherein starting materials are used wherein the groups $R^1$ and $R^2$ are identical since a less complex reaction mixture will be obtained. The process according to the present invention is of special interest for the co-production of acetic acid and ethyl acetate from methyl acetate according to the equation:

$$2\ CH_3COOCH_3 + 2\ CO + 2\ H_2 \rightarrow CH_3COOC_2H_5 + 2\ CH_3COOH$$

since the products can be obtained with high selectivity and close to the stoichiometrically expected ratio. This is of special interest when the process according to the invention is part of an integrated process, wherein acid produced—for instance acetic acid—is to be recycled in the process. Moreover, the process according to the present invention can be carried out conveniently at surprisingly low pressures, e.g. pressures well below 100 bar can be used advantageously.

Suitable starting materials which can be used conveniently in the process according to the present invention include compounds of the general formula $R^1—COOR^2$ and/or $R^3OR^4$, wherein each of $R^1$, $R^2$, $R^3$ and $R^4$, which may be the same or different, represents an alkyl group having from 1 to 12 carbon atoms, or an aryl, alkaryl or aralkyl group having up to 12 carbon atoms, whilst $R^1$ may also represent a hydrogen atom. Preference is given to the use of compounds of the general formula $R^1—COOR^2$ and/or $R^3OR^4$, wherein $R^1$, $R^2$, $R^3$ and $R^4$ are the same and each represents an alkyl group having from 1 to 12 carbon atoms or an aryl, alkaryl or aralkyl group having up to 12 carbon atoms. Most preferred starting materials are methyl acetate and dimethyl ether.

When ethers of the general formula $R^3OR^4$ are used as starting materials in the process according to the present invention, it would appear that these compounds will be converted primarily into the corresponding esters by the introduction of a carbon monoxide moiety into the molecule which molecule may then undergo the homologation reaction according to the present invention. If desired, the reaction according to the present invention may be carried out in two stages when an ether is used as the starting material. Firstly, the ether is converted into the corresponding ester which in its turn, in the same or in a different vessel, is converted into the final products. If desired, mixtures of carboxylic acid esters and/or ethers can be used as starting materials.

Ruthenium compounds which can be used conveniently in the process according to the present invention include ruthenium (III) chloride, ruthenium (III) chloride trihydrate, ruthenium (IV) chloride, ruthenium (III) bromide, the ruthenium oxides, organic ruthenium salts such as ruthenium (III) propionate, ruthenium (III) butyrate, ruthenium pentacarbonyl, triruthenium-dodecacarbonyl and mixed ruthenium halocarbonyls such as bis-(rutheniumtricarbonyl-dibromide), and other organoruthenium complexes.

Further Group VIII metal compounds which can be used together with a ruthenium compound in the catalytic system include palladium and, especially, rhodium compounds, although other Group VIII metal compounds can also be used. Examples of suitable rhodium compounds include rhodium oxide, rhodium (III) hydroxide, rhodium (III) chloride, rhodium (III) chloride trihydrate, rhodium (III) bromide, rhodium (III) iodide and the corresponding pyridine and phosphine complexes such as tris(pyridine) rhodium (III) chloride or dichloro bis-(triphenylphosphine) rhodium, rhodium (III) formate, rhodium (III) acetate, rhodium (III) butyrate, rhodium (III) naphthenate, dirhodium octacarbonyl, tetrarhodium dodecacarbonyl, hexarhodium hexadecacarbonyl, rhodium dicarbonylacetylacetonate and other organo-rhodium complexes. Preference is given to the use of rhodium (III) chloride trihydrate.

Examples of suitable palladium compounds include palladium chloride, palladium chloride dihydrate, palladium bromide, palladium iodide, palladium oxide, or an organic palladium salt or complex such as palladium formate, palladium acetate, palladium butyrate and palladium acetylacetonate. Preferred palladium compounds are palladium chloride, palladium chloride dihydrate and palladium acetate.

The molar ratio of ruthenium compound to further Group VIII metal compound is not critical and can vary between wide limits, e.g. atomic ratios of ruthenium to further Group VIII metal between 50:1 and 1:20, especially 10:1 and 1:5, are suitable.

The amount of ruthenium compound and further Group VIII metal compound to be used is not critical and any amount which exerts catalytic activity can be used. Amounts as flow as 0.001 %w, calculated on carboxylic acid ester or ether to be converted can be used, preference being given to amounts in the range of from 0.01—10 %w, most preferably between 0.05—5 %w.

Any iodide or bromide $R^5Hal$ or $R^5COHal$ may be used in the process according to the present invention, but preferably $R^5$ has one of the preferred meanings given above for $R^2$, and preferably the group $R^5$ is identical to one of the groups $R^1$, $R^2$, $R^3$ or $R^4$ in the starting material, as this avoids the formation of additional mixed products. Especially preferred is the use of a reaction mixture in which $R^1$ and $R^2$ are the same, and the iodide or bromide has the formula $R^2I$, $R^2Br$, $R^2COI$ or $R^2COBr$. Thus for example when using the preferred feedstocks methyl acetate and/or dimethyl ether, methyl iodide or bromide or acetyl iodide or bromide, or any mixture thereof, is preferably used.

The quantity of iodide or bromide added to the reaction mixture is not crucial. Suitably the number of moles of added iodide plus bromide per gram atom of total Group VIII metal is in the range of from 0.1:1 to 200:1, preferably 1:1 to 100:1, and especially 10:1 to 50:1.

As stated hereinbefore, it has most surprisingly been found that a compound according to the general formula $R^5Hal$ and/or $R^5COHal$ can be used instead of the Group II or transition metal iodide or bromide provided that the reaction is carried out in the presence of a phosphine to an amount not more than 2 mol of phosphine per mol of ruthenium. It should be noted that in the Comparative Example D of European Patent Application No. 31606 a phosphine/ruthenium ratio of more than 10 is disclosed which led to a product composition wherein only a trace of ethyl acetate could be detected, the main products being acetic acid and ethylidene diacetate. Care should therefore be taken that the reaction mixture does contain one or more phosphines but in an amount not exceeding 2 mol of phosphine per gram atom of ruthenium, and preferably not more than 1 mol of phosphine per gram atom of ruthenium.

Although the use of a phosphine in a phosphine/ruthenium ratio above 2 does still lead to the production of homologues, it was found that other products appeared to be formed with a rapidly increasing rate, acetic acid anhydride and ethylidene diacetate becoming the predominant (by)-products.

Examples of phosphines which may be present include tertiary phosphines of the general formula $PR^5R^6R^7$, wherein each of $R^5$, $R^6$ and $R^7$, which may be the same or different, represents an alkyl, cycloalkyl or aryl-group having up to 10 carbon atoms. Also phosphines containing two or more trivalent phosphorus atoms as well as phosphites according to the general formula $P(OR^5)(OR^6)(OR^7)$ wherein $R^5$, $R^6$ and $R^7$ have the meanings given hereinabove, can be used. Examples of such compounds are, for instance, tetraphenyldimethylene diphosphine (diphos) and tetraphenyltrimethylenediphosphine. Preferred phosphines comprise trimethylphosphine, triethylphosphine, tri-n-butylphosphine and triphenylphosphine.

The process according to the present invention can be carried out using a wide range of temperatures. Temperatures up to 300°C can be suitably applied. Preference is given to temperatures in the range of from 50°C to 200°C, most preferred temperatures are in the range between 125°C and 175°C.

The process according to the present invention can be carried out using low pressures, e.g. pressures as low as 5 bar. Pressures in the range of from 20 to 100 bar are preferred. Higher pressures, e.g. pressures as high as 1000 bar can be applied, but they are generally not economical because of the investment and energy costs involved.

According to the reaction equation carbon monoxide and hydrogen are consumed in a molar ratio of 1:1. It has been found, however, that without any substantial disadvantage wider molar ratios, e.g. ratios of from 1:10 to 10:1 can be applied. Preference is given to ratios carbon monoxide: hydrogen in the range of from 1:0.5 to 1:3.

The process according to the present invention may be carried out in the presence of a solvent. Suitable solvents include carboxylic acids such as acetic acid or propanoic acid; carboxylic acid esters, such as methyl acetate, ethyl acetate, methylpropionate or ethyl propionate (being used as solvent as well as starting material), and cyclic ethers such as tetrahydrofuran, 1,4-dioxane, 1,3-dioxane and the dioxolanes. Also dialkyl ethers used in excess as starting material may be regarded as solvent for the process according to the present invention. Suitable dialkylethers include dimethyl ether, diethyl ether and methyl t-butyl ether.

Other compounds which can be used as solvent in the process according to the present invention include sulphones and sulphoxides. Examples of such compounds are dimethylsulphone, sulpholane, 2-methyl sulpholane, 3-methyl sulpholane, dimethylsulphoxide and diethyl sulphoxide.

Especially good results are obtained when alkanoic acids such as acetic acid are used as solvent. If however a solvent other than an alkanoic acid is used, it may be desirable to carry out the reaction in the presence of small amounts of a strong acid. For example amounts of strong acid of up to 100 equivalents of acid per gram atom of total Group VIII metal, may be added.

Suitable strong acids include those which in aqueous solution at 20°C have a pKa of less than 3.5, for example organic acids such as *p*-toluene sulphonic acid or trifluoromethane sulphonic acid, or mineral acids such as hydrochloric, sulphuric or perchloric acid.

It has been found that the mild conditions according to the present invention even tolerate the presence of some water in the reaction medium. Although the presence of water is not preferred, amounts of up to 15 %w, based on total solvent, can be present.

The process according to the present invention can be carried out in the liquid phase or in the gaseous phase. Preference is given to a liquid phase which enables a convenient introduction of carbon monoxide and hydrogen into the reaction vessel. If desired, the carbon monoxide and hydrogen can be introduced together into the reaction vessel. The process according to the present invention can be carried out batchwise, semi-continuously or continuously.

The process according to the present invention is also of interest in that it can be integrated with known processes, either for the production of the starting materials (i.e. carboxylic acid esters or the corresponding ethers) or for the conversion of the carboxylic acid esters produced into other products, e.g. by transesterification processes. For instance, when the present process produces ethyl acetate, it can be integrated with a process for the preparation of methyl acetate from acetic acid and methanol using an acidic catalyst. Since the present process produces acetic acid, that compound may be recycled to serve as feedstock for the preparation of methyl acetate. If desired, the present process can also be integrated with a transesterification process, wherein ethylacetate is transesterified with methanol to give methyl acetate (which can be recycled to serve as feedstock for the present process) and ethanol which can either be sold as such or converted into other products such as ethylene. In such a case acetic acid and/or methyl acetate can be removed from the system in an amount equimolar with ethanol produced.

The following Examples illustrate the invention.

Example I

The experiment was carried out in a 300 ml magnet-driven autoclave of Hastelloy C (Trade Mark) which contained 25 ml methyl acetate, 25 ml acetic acid, 60 mmol methyl iodide, 0.5 mmol rhodium (III) chloride trihydrate, 1 mmol ruthenium (III) chloride trihydrate, and 1 mmol triphenylphosphine. The vessel was flushed with carbon monoxide, and then pressurised with carbon monoxide (20 bar partial pressure) and hydrogen (20 bar partial pressure). The autoclave was then heated to 160°C and kept at this temperature for 5 hours, during which time the pressure was maintained constant by feeding in carbon monoxide and hydrogen (1:1) as required. After this time the reaction mixture was analysed by gas-liquid chromatography and shown to contain 10.4 %w ethyl acetate. On a molar basis the conversion of the starting material was about 40% with an almost 100% selectivity towards the two products ethyl acetate and acetic acid. Only traces (less than 0.5%) of by-products were detected: in particular, no alcohols were detected.

Example II

The method of Example I was repeated except that only 0.25 mmol rhodium (III) chloride trihydrate and 0.5 mmol triphenylphosphine were used. After the reaction time was over, the reaction mixture contained 10.6 %w ethyl acetate, and 45% of the methyl acetate had been converted to the desired products. Only traces of by-products were observed.

Example III

The method of Example II was repeated except that 1 mmol triphenylphosphine was used at an initial carbon monoxide pressure of 15 bar and hydrogen pressure of 30 bar. 80% of the methyl acetate was converted into products, and the reaction mixture contained 18.9 %w ethyl acetate. Only traces of by-products were observed.

**Claims**

1. A process for the co-production of carboxylic acids of the general formula $R^1$—COOH and $R^2$—COOH and carboxylic acid esters of the general formula $R^1$—$COOCH_2R^2$ and $R^2$—$COOCH_2R^1$, wherein each of the groups $R^1$ and $R^2$, which may be the same or different, represents an alkyl group having from 1 to 20 carbon atoms which may be substituted by fluorine or chlorine, or an aryl, alkaryl or aralkyl group which may be substituted by fluorine or chlorine whilst $R^1$ may also represent a hydrogen atom, in which process a carboxylic acid ester of the general formula $R^1$—$COOR^2$ and/or an ether of the general formula $R^3OR^4$ wherein $R^1$ and $R^2$ are as defined hereinbefore and each of $R^3$ and $R^4$, which may be the same or different, represents an alkyl group having from 1 to 20 carbon atoms which may be substituted by fluorine or chlorine or an aryl, alkaryl or aralkyl group which may be substituted by fluorine or chlorine is reacted with carbon monoxide and hydrogen at elevated temperature and pressure characterized in that the reaction is carried out in the presence of a catalytic system which comprises a ruthenium compound, a further Group VIII metal compound except iron, nickel and cobalt, a phosphine and a compound of the general formula $R^5Hal$ or $R^5COHal$ where $R^5$ has one of the meanings given above for $R^2$ and Hal represents an iodine or bromine atom, the reaction mixture being substantially free from Group II metal iodides or bromides, and containing not more than 2 mol of phosphine per gram atom of ruthenium.

2. A process according to claim 1, characterized in that as ruthenium compound is used ruthenium (III) chloride, ruthenium (III) chloride trihydrate, ruthenium (IV) chloride, ruthenium (III)

bromide, ruthenium oxide or an organic ruthenium salt or complex.

3. A process according to claim 1 or 2 characterized in that as further Group VIII metal compound is used a rhodium compound such as rhodium oxide, rhodium (III) hydroxide, rhodium (III) chloride, rhodium (III) chloride trihydrate, rhodium (III) bromide, rhodium (III) iodide or an organic rhodium salt or complex, preferably rhodium (III) chloride trihydrate, or a palladium compound such as palladium chloride, palladium chloride dihydrate, palladium bromide, palladium iodide, palladium oxide or an organic palladium salt or complex, preferably palladium chloride, palladium chloride dihydrate or palladium acetate.

4. A process according to any one of the preceding claims, in which a phosphine is present in the reaction mixture in an amount of not more than 1 mol per gram atom of ruthenium.

5. A process according to claim 4, in which tertiary phosphines according to the general formula $PR^5R^6R^7$ or phosphines containing two or more phosphorus atoms or phosphites according to the general formula $P(OR^5)(OR^6)(OR^7)$ are used, wherein each of $R^5$, $R^6$ and $R^7$, which may be the same or different, represents an alkyl, cycloalkyl or aryl group having up to 10 carbon atoms, preferably triphenylphosphine.

6. A process according to any one of the preceding claims characterized in that the reaction is carried out at a temperature in the range of from 50°C to 200°C, and especially between 125°C and 175°C.

7. A process according to any one of the preceding claims characterized in that the process is carried out using a pressure between 20 and 100 bar.

**Patentansprüche**

1. Ein Verfahren zur gleichzeitigen Herstellung von Carbonsäuren der allgemeinen Strukturformeln $R^1$—COOH und $R^2$—COOH und von Carbonsäureestern der allgemeinen Strukturformeln $R^1—COOCH_2R^2$ und $R^2—COOCH_2R^1$, in welchen jede der beiden Gruppen $R^1$ und $R^2$, die gleich oder verschieden sein können, eine Alkylgruppe mit 1 bis 20 Kohlenstoffatomen, die durch FLuor oder Chlor substituiert sein kann, oder eine Aryl-, Alkaryl- oder Aralkylgruppe, die durch Fluor oder Chlor substituiert sein können, darstellt, wobei $R^1$ auch ein Wasserstoffatom bedeuten kann, in welchem Verfahren ein Carbonsäureester der allgemeinen Formel $R^1—COOR^2$ und/oder ein Äther der allgemeinen Formel $R^3OR^4$, in welchen $R^1$ und $R^2$ wie oben definiert sind und jede der beiden Gruppen $R^3$ und $R^4$, welche gleich oder verschieden sein können, eine Alkylgruppe mit 1 bis 20 Kohlenstoffatomen, welche durch Fluor oder Chlor substituiert sein kann oder eine Aryl-, Alkaryl- oder Aralkylgruppe, welche durch Fluor oder Chlor substituiert sein kann, darstellt, mit Kohlenmonoxid und Wasserstoff bei erhöhter Temperatur und erhöhtem Druck umgesetzt wird, dadurch gekennzeichnet, daß die Umsetzung in Gegenwart eines katalytischen Systems, welches eine Rutheniumverbindung, eine weitere Verbindung eines Metalls der Gruppe VIII, mit Ausnahme von Eisen, Nickel und Kobalt, ein Phosphin und eine Verbindung der allgemeinen Formel $R^5$Hal oder $R^5$COHal, wobei $R^5$ eine der vorstehend für $R^2$ angegebenen Bedeutungen hat, und Hal ein Jod- oder Bromatom bedeutet, enthält, wobei die Reaktionsmischung im wesentlichen frei ist von Metalliodiden oder -bromiden der Gruppe II, und nicht mehr als 2 Mol Phosphin pro Grammatom Ruthenium enthält, durchgeführt wird.

2. Ein Verfahren gemäß Anspruch 1, dadurch gekennzeichnet, daß als Rutheniumverbindung Ruthenium(III)chlorid, Ruthenium(III)chlorid-trihydrat, Ruthenium(IV)chlorid, Ruthenium(III)-bromid, Rutheniumoxid oder ein organisches Rutheniumsalz oder ein organischer Rutheniumkomplex eingesetzt werden.

3. Ein Verfahren gemäß Anspruch 1 oder 2, dadurch gekennzeichnet, daß als weitere Verbindung eines Metalls der Gruppe VIII eine Rhodiumverbindung, wie z.B. Rhodiumoxid, Rhodium(III)hydroxid, Rhodium(III)chlorid, Rhodium(III)chlorid - trihydrat, Rhodium(III)-bromid, Rhodium(III)iodid oder ein organisches Rhodiumsalz oder ein organischer Rhodiumkomplex, vorzugsweise Rhodium(III)chlorid-trihydrat, oder eine Palladiumverbindung, wie z.B. Palladiumchlorid, Palladiumchlorid-dihydrat, Palladiumbromid, Palladiumiodid, Palladiumoxid oder ein organisches Palladiumsalz oder ein organischer Palladiumkomplex, vorzugsweise Palladiumchlorid Palladiumchlorid-dihydrat oder Palladiumacetat, eingesetzt werden.

4. Ein Verfahren gemäß irgendeinem der vorstehenden Ansprüche, in welchem ein Phosphin in einer Menge von nicht mehr als 1 Mol pro Grammatom Ruthenium in der Reaktionsmischung vorhanden ist.

5. Ein Verfahren gemäß Anspruch 4, in welchem tertiäre Phosphine mit der allgemeinen Formel $PR^5R^6R^7$ oder Phosphine, enthaltend 2 oder mehr Phosphoratome, oder Phosphite der allgemeinen Formel $P(OR^5)(OR^6)(OR^7)$ verwendet werden, wobei jede der Gruppen $R^5$, $R^6$ und $R^7$, welche gleich oder verschieden sein können, eine Alkyl-, Cycloalkyl-, oder Arylgruppe mit bis zu 10 Kohlenstoffatomen, darstellt, vorzugsweise Triphenylphosphin.

6. Ein Verfahren gemäß irgendeinem der vorstehenden Ansprüche, dadurch gekennzeichnet, daß die Reaktion bei einer Temperatur im Bereich von 50°C bis 200°C, und vorzugsweise von 125°C bis 175°C, durchgeführt wird.

7. Ein Verfahren gemäß irgendeinem der vorstehenden Ansprüche, dadurch gekennzeichnet, daß das Verfahren bei einem Druck zwischen 20 und 100 bar durchgeführt wird.

**Revendications**

1. Un procédé pour la coproduction d'acides

carboxyliques des formules générales $R^1$—COOH et $R^2$—COOH et d'esters d'acides carboxyliques des formules générales $R^1$—$COOCH_2R^2$ et $R^2$—$COOCH_2R^1$, où les groupes $R^1$ et $R^2$, qui peuvent être identiques ou différentes, représentent chacun un groupe alcoyle ayant de 1 à 20 atomes de carbone qui peut être substitué par du fluor ou du chlore ou un groupe aryle, alcaryle ou aralcoyle qui peut être substitué par du fluor ou du chlore, tandis que $R^1$ peut aussi représenter un atome d'hydrogène, procédé dans lequel un ester d'acide carboxylique de la formule générale $R^1$—$COOR^2$ et/ou un éther de la formule générale $R^3OR^4$, où $R^1$ et $R^2$ sont tels que définis ci-dessus et $R^3$ et $R^4$, qui peuvent être identiques ou différents, représentent chacun un groupe alcoyle ayant de 1 à 20 atomes de carbone qui peut être substitué par du fluor ou du chlore ou un groupe aryle, alcaryle ou aralcoyle qui peut être substitué par du fluor ou du chlore, sont mis à réagir avec de l'oxyde de carbone et de l'hydrogène à température et pression élevées, caractérisé en ce que la réaction est conduite en présence d'un système catalytique qui comprend un composé du ruthénium, un composé d'un autre métal du groupe VIII à l'exception du fer, du nickel et du cobalt, une phosphine et un composé de la formule générale $R^5$Hal ou $R^5$COHal où $R^5$ a une des significations indiquées ci-dessus pour $R^2$ et Hal représente un atome d'iode ou de brome, le mélange réactionnel étant sensiblement exempt d'iodures ou de bromures de métaux du groupe II et ne contenant pas plus de 2 moles de phosphine par atome-gramme de ruthénium.

2. Un procédé selon la revendication 1, caractérisé en ce que comme composé du ruthénium on utilise du chlorure de ruthénium (III), du trihydrate de chlorure de ruthénium (III), du chlorure de ruthénium (IV), du bromure de ruthénium (III) de l'oxyde de ruthénium ou un sel organique ou un complexe de ruthénium.

3. Un procédé selon la revendication 1 ou 2, caractérisé en ce que comme composé d'un autre métal du groupe VIII on utilise un composé du rhodium tel que l'oxyde de rhodium, l'hydroxyde de rhodium (III), le chlorure de rhodium (III), le trihydrate de chlorure de rhodium (III), le bromure de rhodium (III), l'iodure de rhodium (III) ou un sel organique ou un complexe de rhodium, de préférence le trihydrate de chlorure de rhodium (III), ou un composé du palladium tel que le chlorure de palladium, le dihydrate de chlorure de palladium, le bromure de palladium, l'iodure de palladium, l'oxyde de palladium ou un sel organique ou un complexe de palladium, de préférence le chlorure de palladium, le dihydrate de chlorure de palladium ou l'acétate de palladium.

4. Un procédé selon l'une quelconque des revendications précédentes, dans lequel une phosphine est présente dans le mélange réactionnel à raison de pas plus de 1 mole par atome-gramme de ruthénium.

5. Un procédé selon la revendication 4, dans lequel on utilise des phosphines tertiaires de la formule générale $PR^5R^6R^7$ ou des phosphines contenant deux atomes de phosphore ou plus ou des phosphites de la formule générale $P(OR^5)(OR^6)(OR^7)$, où $R^5$, $R^6$ et $R^7$, qui peuvent être identiques ou différentes, représentent chacun un groupe alcoyle, cycloalcoyle ou aryle ayant jusqu'à 10 atomes de carbone, de préférence la triphénylphosphine.

6. Un procédé selon l'une quelconque des revendications précédentes, caractérisé en ce que la réaction est conduite à une température comprise entre 50°C et 200°C et spécialement entre 125°C et 175°C.

7. Un procédé selon l'une quelconque des revendications précédentes, caractérisé en ce qu'on met en oeuvre le procédé en utilisant une pression comprise entre 20 et 100 bars.